(19) Europäisches Patentamt
European Patent Office
Office européen des brevets

(11) Numéro de publication: **0 217 700 B1**

(12) # FASCICULE DE BREVET EUROPEEN

(45) Date de publication de fascicule du brevet: **30.03.94**

(21) Numéro de dépôt: **86401928.6**

(22) Date de dépôt: **02.09.86**

(51) Int. Cl.5: **C07D 401/14**, C07D 405/14, C07D 409/14, C07D 471/04, A61K 31/415, A61K 31/44, A61K 31/505

(54) **Derives de benzimidazole, leur preparation et leur application en therapeutique.**

(30) Priorité: **11.09.85 FR 8513453**

(43) Date de publication de la demande:
**08.04.87 Bulletin 87/15**

(45) Mention de la délivrance du brevet:
**30.03.94 Bulletin 94/13**

(84) Etats contractants désignés:
**AT BE CH DE FR GB IT LI LU NL SE**

(56) Documents cités:
**EP-A- 0 099 139**
**EP-A- 0 144 101**
**EP-A- 0 151 824**
**EP-A- 0 151 826**
**US-A- 4 281 005**

(73) Titulaire: **SYNTHELABO**
**22, Avenue Galilée**
**F-92350 Le Plessis Robinson(FR)**

(72) Inventeur: **Manoury, Philippe**
**L'Orée de Verrières**
**38, Avenue des Vaupépins**
**F-91370 Verrieres le Buisson(FR)**
Inventeur: **Binet, Jean**
**12 Hameau de la Gondole**
**F-91650 Breuillet(FR)**
Inventeur: **Defosse, Gérard**
**29, rue de Tolbiac**
**F-75013 Paris(FR)**

(74) Mandataire: **Thouret-Lemaitre, Elisabeth et al**
**SYNTHELABO,**
**Service Brevets,**
**B.P. 72**
**F-92352 Le Plessis-Robinson Cédex (FR)**

EP 0 217 700 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention a pour objet des dérivés de benzimidazole, leur préparation et leur application en thérapeutique.

Certains auteurs ont déjà décrit des dérivés de benzimidazole, respectivement d'imidazopyridine (EP-A-0099139 EP-A-0144101 ; EP-A 0151826 ; EP-A-0151824 ; US-A-4281005).

Les composés de l'invention se différencient principalement de ceux qui sont décrits dans l'art antérieur par le fait que le modèle de substitution du radical 4-aminopipéridinyle est différent par la position des substituants benzimidazole, respectivement imidazopyridine et 2-pyrimidine dans la molécule.

Les composés de l'invention répondent à la formule (I) donnée dans l'annexe 1 dans laquelle
X est CH ou N,
$R_1$ est soit un atome d'hydrogène, soit un radical benzyle pouvant porter de 1 à 3 substituants choisis parmi les atomes d'halogène, les radicaux trifluorométhyle, $(C_{1-4})$alkyles, $(C_{1-4})$alcoxy, cyano, méthylthio, méthylsulfinyle et méthylsulfonyle, soit un radical hétérocycle-méthyle dans lequel l'hétérocycle peut être un radical pyridinyle, thiényle ou furannyle,
$R_2$ est un atome d'hydrogène ou un radical $(C_{1-4})$alkyle,
$R_3$ est un atome d'hydrogène ou le radical hydroxy,
$R_4$ est un atome d'hydrogène ou un radical $(C_{1-4})$alkyle.

Les sels d'addition que forment les composés (I) avec les acides pharmaceutiquement acceptables font partie de l'invention.

Les composés préférés de l'invention sont ceux dans lesquels X est CH ou N, $R_1$ est un radical fluoro-4 benzyle et $R_2$, $R_3$ et $R_4$ ont les significations données dans la revendication 1.

Parmi les composés de l'invention dans lesquels X est CH, les composés de choix sont ceux dans lesquels $R_1$ est un radical benzyle portant un ou deux substituants, et plus particulièrement ceux qui portent en position 4 un seul substituant qui est un atome de fluor ou de chlore ou le radical méthyle, méthoxy, méthylthio, trifluorométhyle, cyano ou méthylsulfinyle.

Lorsque $R_3$ est OH et $R_4$ est H, les formes tautomères des composés font partie de l'invention (voir annexe 1).

Selon l'invention on peut préparer les composés (I) selon les deux schémas réactionnels principaux donnés en annexe 2, dont les méthodes A, B, C, D sont exemplifiées ci-dessous.

La méthode C, la plus générale, s'applique à tous les composés, tandis que la méthode D n'est appliquable qu'aux composés dans lesquels $R_2$ est différent de H. Les méthodes A et B ne sont appliquables que lorsque $R_1$ est différent de H.

Le procédé de l'invention consiste à faire réagir un composé de formule (II) avec une $(R_2)(R)$amino-4 pipéridine de formule

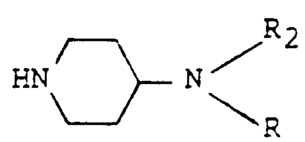

dans laquelle $R_2$ = H ou $(C_{1-4})$alkyle et R représente
. soit un atome d'hydrogène,
. soit un groupe $(C_{1-4})$alcoxycarbonyle,
. soit le groupe

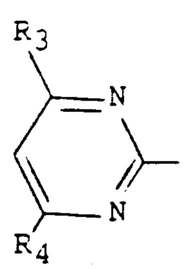

($R_3$ = H ou OH, $R_4$ = H ou $(C_{1-4})$alkyle, et,

. lorsque R est H on introduit le groupe pyrimidinyle dans un second temps,
. lorsque R est un groupe $(C_{1-4})$alcoxycarbonyle on élimine ce groupe par hydrolyse puis on introduit le groupe pyrimidinyle.

Selon la méthode A, on condense un composé (II) avec une $(R_2)$(alcoxycarbonyl)amino-pipéridine (III) par chauffage à 150 °C environ, puis on hydrolyse le composé (IV) obtenu à l'aide d'acide bromhydrique en milieu acétique en composé (V) que l'on fait réagir avec une pyrimidine (VI), dans laquelle Y est un groupe partant tel que $SCH_3$, Cl, Br ou I, en présence ou non d'un solvant, à une température de 50 à 200 °C.

Selon la méthode B, on fait réagir un composé (II) avec une $(R_2)$amino-pipéridine, en présence de carbonate de potassium dans un solvant alcoolique, puis on condense le composé (V) avec un pyrimidine (VI), dans laquelle Y est un groupe partant tel que $SCH_3$, Cl, Br ou I, en présence ou non d'un solvant, à une température de 50 à 200 °C.

Selon la méthode C, on fait réagir un composé (II) avec un composé (VIII) (obtenu par alkylation d'une benzyl-1 ou éthoxycarbonyl-1 amino-4 pipéridine avec une halogéno-2 ou alkylthio-2 pyrimidine (VI), suivie d'une débenzylation catalytique ou d'une hydrolyse pour éliminer le groupe protecteur en position 1), dans un solvant alcoolique, à la température du reflux.

Selon la méthode D, on part d'un composé (II), dans lequel $R_1$ est H, que l'on condense avec une $(R_2)$-(alcoxycarbonyl)aminopipéridine (III) par chauffage à 150 °C, on alkyle le composé obtenu (X) par action d'un halogénure d'alkyle pour obtenir le composé (IV) que l'on hydrolyse, puis on condense le composé (V) avec une pyrimidine (VI) comme selon la méthode A.

Dans les schémas réactionnels représentés en annexe 2 les radicaux ont les significations données ci-dessus.

Les composés de formules (II, avec X = CH), (VI) et (VII) sont décrits dans la littérature ; les composés de formules (II, avec X = N), (III), (IV), (V) et (VIII, avec $R_3$ = OH) sont nouveaux.

Les exemples suivants illustrent l'invention. Les analyses et les spectres IR et RMN confirment la structure des composés.

Exemple 1. [[[[(fluoro-4 phényl)méthyl]-1 1H-benzimidazolyl-2]-1 pipéridinyl-4]méthylamino]-2 pyrimidinol-4. (Méthodes A et B, X = CH, $R_1$ = 4F-$C_6H_4$-$CH_2$, $R_2$ = $CH_3$, $R_3$ = OH, $R_4$ = H).

1.1. [[(Fluoro-4 phényl)méthyl-1] 1H-benzimidazolyl-2]-1 N-méthyl-pipéridinamine-4 (méthode A).

1.1.1. On solubilise 19 g (0,09 mole) de chlorhydrate de pipéridinyl-4 carbamate d'éthyle dans 120 ml de méthanol et neutralise avec 17,2 ml de méthylate de sodium 5,3 N. On filtre, évapore à sec. On mélange alors le résidu d'évaporation avec 21,6 g (0,083 mole) de chloro-2 [(fluoro-4 phényl) méthyl]-1 1H-benzimidazole et chauffe à 140 °C pendant 5 heures. On reprend la masse réactionnelle avec du chlorure de méthylène, alcalinise avec de la soude 2N. On lave la phase organique avec de l'eau, sèche, filtre, évapore. On chromatograhie l'huile obtenue sur silice (éluant : chlorure de méthylène-méthanol 97,5/2,5). On obtient le [[[(fluoro-4 phényl)méthyl]-1 1H-benzimidazolyl-2]-1 pipéridinyl-4] carbamate d'éthyle.
F = 136 °C.

1.1.2. A une suspension refroidie au bain de glace, de 3,3 g (0,068 mole) d'hydrure de sodium à 50 % dans 40 ml de diméthylformamide, on ajoute goutte à goutte en une demi-heure environ, 21,6 g (0,054 mole) du composé obtenu précédemment en solution dans 40 ml de diméthylformamide. On laisse le mélange revenir à la température ambiante et agite 2 h. On refroidit de nouveau à l'aide d'un bain d'eau glacée, et ajoute 4,7 ml (0,075 mole) d'iodure de méthyle (d = 2,28) en solution dans 30 ml de DMF. On laisse la température revenir à 20 °C et agite 1 h. On verse le mélange réactionnel dans un mélange d'eau, d'hexane et d'éther isopropylique et agite jusqu'à cristallisation. On filtre le précipité et sèche. On obtient le [[[(fluoro-4 phényl)méthyl]-1 1H-benzimidazolyl-2]-1 pipéridinyl-4]N-méthyl carbamate d'éthyle.
F = 125 °C.

1.1.3. On porte à la température du reflux, pendant 1,5 h, 7 g (0,017 mole) du composé obtenu précédemment, en solution dans 140 ml d'acide acétique et 140 ml d'acide bromhydrique à 48 %. On évapore à sec, reprend avec de l'eau, alcalinise avec de la soude 2N. On ajoute de l'éther et agite jusqu'à ce que le produit cristallise. On obtient ainsi le monohydrate de [[(fluoro-4 phényl)méthyl]-1 1H-benzimidazolyl-2]-1 N-méthyl pipéridinamine-4.
F = 50 °C.

1.2. [[(Fluoro-4 phényl)méthyl]-1 1H-benzimidazolyl-2]-1-N-méthyl pipéridinamine-4 (méthode B).
On porte à la température du reflux, pendant 192 h, 8,7 g (0,05 mole) de méthylamino-4-pipéridine (sous forme d'acétate), 13 g (0,05 mole) de (fluoro-4-benzyl)-1 chloro-2 benzimidazole et 13,8 g (0,1 mole) de

carbonate de potassium dans 250 ml d'alcool isoamylique.
On refroidit le mélange, l'évapore à sec. On reprend le résidu avec un mélange d'eau et d'éther, et agite jusqu'à cristallisation. On filtre le composé (V) obtenu sous forme d'hydrate. On reprend le précipité avec du toluène, l'agite jusqu'à dissolution et sèche la solution avec du sulfate de magnésium, filtre et évapore. L'huile résiduelle est triturée dans de l'éther de pétrole. On filtre le produit solide et le sèche. On obtient ainsi le composé que fond à 77-80 °C.
1.3. [[[[(Fluoro-4 phényl)méthyl]-1 1H-benzimidazolyl-2]-1 pipéridinyl-4]méthylamino]-2 pyrimidinol-4.
On chauffe, à 170 °C, pendant 10 h, 2,5 g (0,007 mole) du composé obtenu sous 1.1. et 1 g (0,007 mole) de méthylthiouracile. Après refroidissement la masse réactionnelle est chromatographiée sur une colonne de silice (éluant = dichlorométhane/méthanol 97/3). On obtient le composé que l'on fait recristalliser dans de l'éthanol.
F = 217 °C.

Exemple 2. [[(Fluoro-4 phényl)méthyl]-1 1H-benzimidazolyl-2]-1 N-pyrimidinyl-2)-pipéridinamine-4.
(Méthode A, $R_1$ = 4F-$C_6H_4$-$CH_2$, $R_2$ = $R_3$ = $R_4$ = H, X = CH).

2.1. [[(Fluoro-4 phényl)méthyl]-1 1H-benzimidazolyl-2]-1 pipéridinamine-4.
On porte à la température du reflux, pendant 2 h, 14,5 g de [[[(fluoro-4 phényl)méthyl]-1 1H-benzimidazolyl-2]-1 pipéridinyl-4 carbamate d'éthyle en solution dans 250 ml d'acide acétique et 250 ml d'acide bromhydrique à 48 %. On évapore à sec, reprend avec de l'eau, alcalinise avec de la soude 2N, filtre le précipité, le lave avec de l'eau et le sèche. On recueille le produit sous forme monohydratée.
2.2. [[(Fluoro-4 phényl)méthyl]-1 1H-benzimidazolyl-2]-1 N-pyrimidinyl-2)-pipéridinamine-4.
On porte à la température du reflux, pendant 2 jours et 2 nuits, un mélange de 1,71 g (0,005 mole) de [[-(fluoro-4 phényl)-méthyl]-1 1H-benzimidazolyl-2]-1 pipéridinamine-4 0,57 g (0,005 mole) de chloro-2 pyrimidine et 0,43 g (0,052 mole) de bicarbonate de sodium et 19 ml d'éthanol. On évapore à sec, ajoute de l'eau et de la soude 2N. On reprend par du chlorure de méthylène, décante et lave à l'eau. On chromatographie le produit sur une colonne de silice (éluant dichlorométhane/méthanol 47/3).
On obtient le produit que l'on fait cristalliser dans de l'éther.
F = 190 °C.

Exemple 3. [[[[(Fluoro-4 phényl)méthyl]-1 3H-imidazo[4,5-b] pyridinyl-2]-1 pipéridinyl-4]méthylamino]-2 pyrimidinol-4
(Méthode C ; X = N, $R_1$ = 4F-$C_6H_4$-$CH_2$, $R_2$ = $CH_3$, $R_3$ = OH, $R_4$ = H).

3.1. [(Pipéridinyl-4) (méthyl)amino]-2 1H-pyrimidinone-4. (composé VIII)
3.1.1. [(Ethoxycarbonyl-1 pipéridinyl-4) (méthyl)amino]-2 1H-pyrimidinone-4.
Dans un ballon placé sous circulation d'azote et dont la sortie des vapeurs est reliée à un système de lavage par l'eau de Javel, on place 36 g (0,193 mol) de méthylamino-4 pipéridine-1-carboxylate d'éthyle en présence de 27,44 g (0,193 mol) de S-méthylthiouracile et de 730 ml de xylène, on chauffe à la température du reflux du solvant pendant environ 50 h. Le solvant est ensuite évaporé à siccité sous vide et le produit solide obtenu est dissous dans de l'acétate de butyle à reflux. Cette solution est filtrée à chaud puis le produit, qui recristallise au refroidissement, est essoré et séché. Le composé fond à 177-179 °C.
3.1.2. [(Pipéridinyl-4) (méthyl)amino]-2 1H-pyrimidinone-4.
On chauffe à la température du reflux, pendant 1 h $\frac{1}{4}$, une solution de 19,73 g (0,07 mol), du composé précédent dans 150 ml d'acide bromhydrique à 48 % et 150 ml d'acide acétique. On évapore les acides à siccité au rotavapor. Le résidu est repris par un peu d'eau et réévaporé à siccité, cette opération étant répétée 3 fois.
On reprend finalement le résidu, en refroidissant, par une solution excédentaire de soude concentrée, puis agite la suspension résultante au bain sonique, on la refroidit dans de la glace et l'essore. Le solide est pressé, lavé avec très peu d'eau glacée et rincé ensuite abondamment à l'éther. On obtient un solide blanc.
F = 220-223 °C.
3.2. [[[[(Fluoro-4 phényl)méthyl]-1 3H-imidazo [4,5-b] pyridinyl-2]-1 pipéridinyl-4]méthylamino]-2 pyrimidinol-4.
On chauffe à la température du reflux, pendant 5 h, 1,3 g ($5.10^{-3}$ mole) de chloro-2 [(fluoro-4 phényl)-méthyl]-1 3H-imidazo[4,5-b]pyridine, 1,1 g ($5.10^{-3}$ mole) de [(pipéridinyl-4) (méthyl)amino]-2 1H-pyrimidinone-4 dans 50 ml de méthyl-3 butanol-1. On laisse le mélange une nuit au repos et ajoute 0,7 g

($5.10^{-3}$ mole) de carbonate de potassium. On porte de nouveau à la température de reflux pendant 5 h. On refroidit le mélange et l'évapore à sec. On reprend le résidu d'évaporation avec de l'eau, extrait avec de l'acétate d'éthyle, lave la phase organique avec de l'eau, sèche sur sulfate de magnésium, filtre et évapore. On purifie le produit par chromatographie sur colonne de silice (éluant dichlorométhane/méthanol 95/5) et recueille le produit fondant à 185-187 °C.

Exemple 4. [[[(Cyano-4 phénylméthyl)-1 1H-benzimidazolyl-2]-1 pipéridinyl-4]méthylamino]-2 pyrimidinol-4.

4.1. [[1-H benzimidazolyl-2]-1 pipéridinyl-4] N-méthyl-carbamate de tertiobutyle.
On chauffe à la température du reflux, pendant 4 ½ h, 32,7 g (0,15 mole) de (pipéridinyl-4) N-méthyl carbamate de tertiobutyle, 20,8 g (0,136 mole) de chloro-2 1H-benzimidazole dans 275 ml de méthyl-3 butanol-1.
On évapore le solvant sous vide, et reprend le résidu avec 20 ml de méthanol chaud. On ajoute 28,5 ml de méthylate de sodium 5,3 N et ajoute 200 ml d'eau. On essore le précipité formé, le lave avec de l'eau et le sèche.
On obtient un solide fondant à 242 °C.
4.2. (Cyano-4 phénylméthyl)-1 [1H benzimidazolyl-2]-1 pipéridinyl-4]-N-méthyl carbamate de t-butyle.
A une suspension de 1,14 g (0,0237 mole) de NaH à 50 % dans 30 ml de diméthylformamide, on ajoute par portions 6,04 g (0,02 mole) du produit précédent et agite 1 h après la fin de l'introduction. On refroidit le mélange à 0 °C et ajoute 4,5 g (0,023 mole) de bromométhyl-4 benzonitrile en solution dans 15 ml de diméthylformamide. On agite le mélange 2 h à 0 °C puis le verse dans de l'eau. On extrait avec de l'éther, lave la phase organique avec de l'eau, sèche, filtre et évapore.
On purifie le produit par chromatographie sur une colonne de silice (éluant dichlorométhane/méthanol 98/2). On obtient le produit fondant à 146 °C.
4.3. [(Cyano-4 phénylméthyl)-1 1H-benzimidazolyl-2]-1 N-méthyl pipéridinamine-4.
On chauffe, à 50 °C,pendant 1 h, 4,8 g (0,0107 mole) du produit précédent dans 20 ml d'acide chlorhydrique 3N. Lorsque l'hydrolyse est complète, on refroidit la solution, alcalinise avec de la soude 5N, extrait avec du dichlorométhane. On obtient le produit fondant à 133 °C.
4.4. [[[(Cyano-4 phénylméthyl)-1 1H-benzimidazolyl-2]-1 pipéridinyl-4]méthylamino]-2 pyrimidinol-4.
On porte à la température du reflux, pendant 168 h, 3,5 g (0,01 mole) du produit précédent, 1,4 g (0,01 mole) de S-méthyl-thiouracile dans 35 ml de toluène. On refroidit le mélange, l'évapore à sec et chromatographie le résidu sur une colonne de silice (éluant dichlorométhane/méthanol 98/2 puis 96/4). On obtient le produit fondant à 198 °C.

Les composés de l'invention préparés à titre d'exemples sont représentés dans le tableau suivant (I).
Les composés intermédiaires de formule (IV) qui sont nouveaux sont représentés dans le tableau suivant (II).

TABLEAU I

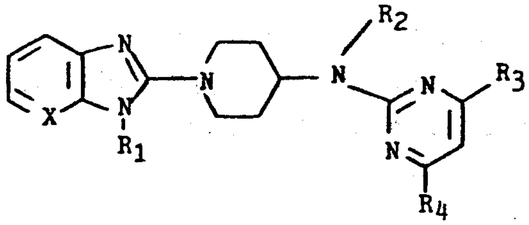

(I)

| N° | X | $R_1$ | $R_2$ | $R_3$ | $R_4$ | F°C |
|---|---|---|---|---|---|---|
| 1 | CH | H | $CH_3$ | OH | H | 280 |
| 2 | CH | $C_6H_5-CH_2$ | $CH_3$ | OH | H | 254 |
| 3 | CH | $4-F-C_6H_4-CH_2$ | H | H | H | 190 |
| 4 | CH | $4-F-C_6H_4-CH_2$ | H | OH | H | 244 |
| 5 | CH | $4-F-C_6H_4-CH_2$ | $CH_3$ | OH | H | 217 |
| 6 | CH | $4-F-C_6H_4-CH_2$ | $CH_3$ | OH | $CH_3$ | 241 |
| 7 | CH | $4-F-C_6H_4-CH_2$ | $CH_3$ | OH | $C_3H_7$ | 247 |
| 8 | CH | $4-F-C_6H_4-CH_2$ | $C_2H_5$ | OH | H | 210 |
| 9 | CH | $4-F-C_6H_4-CH_2$ | $C_3H_7$ | OH | H | 201 |
| 10 | CH | $4-CH_3O-C_6H_4-CH_2$ | $CH_3$ | OH | H | 206 |
| 11 | CH | $4-CH_3-C_6H_4-CH_2$ | $CH_3$ | OH | H | 215 |
| 12 | CH | $4-CF_3-C_6H_4-CH_2$ | $CH_3$ | OH | H | 140 |
| 13 | CH | $4-CN-C_6H_4-CH_2$ | $CH_3$ | OH | H | 198 |
| 14 | CH | $4-Cl-C_6H_4-CH_2$ | $CH_3$ | OH | H | 146 |
| 15 | CH | $4-CH_3S-C_6H_4-CH_2$ | $CH_3$ | OH | H | 215 |
| 16 | CH | $4-CH_3SO-C_6H_4-CH_2$ | $CH_3$ | OH | H | 184 |
| 17 | CH | $4-F-3CF_3-C_6H_3-CH_2$ | $CH_3$ | OH | H | 195 |
| 18 | CH | $2,4diF-C_6H_3-CH_2$ | $CH_3$ | OH | H | 213 |
| 19 | CH | 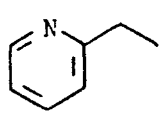 | $CH_3$ | OH | H | 254 |
| 20 | CH | 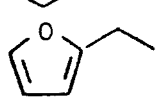 | $CH_3$ | OH | H | 160 |
| 21 | N | $4F-C_6H_4-CH_2$ | $CH_3$ | OH | H | 185 |

TABLEAU II

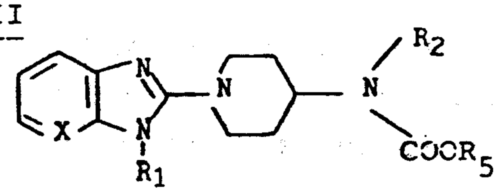

(IV)

| X | $R_1$ | $R_2$ | $COOR_5$ | F°C |
|---|---|---|---|---|
| CH | H | $CH_3$ | $COOC_2H_5$ | 218 |
| CH | H | $CH_3$ | COOtBu | 237 |
| CH | $4\text{-}F\text{-}C_6H_4\text{-}CH_2$ | $CH_3$ | $COOC_2H_5$ | 125 |
| CH | $4\text{-}F\text{-}C_6H_4\text{-}CH_2$ | $C_2H_5$ | $COOC_2H_5$ | 129 |
| CH | $4\text{-}CH_3\text{-}C_6H_5\text{-}CH_2$ | $CH_3$ | $COOC_2H_5$ | 107 |
| CH | $4\text{-}CN\text{-}C_6H_4\text{-}CH_2$ | $CH_3$ | COOtBu | 146 |
| CH | $4\text{-}Cl\text{-}C_6H_4\text{-}CH_2$ | $CH_3$ | $COOC_2H_5$ | 120 |
| CH | $4\text{-}F\text{-}3CF_3\text{-}C_6H_4\text{-}CH_2$ | $CH_3$ | COOtBu | 125 |
| CH | $2,4diF\text{-}C_6H_4\text{-}CH_2$ | $CH_3$ | COOtBu | 158 |
| CH | 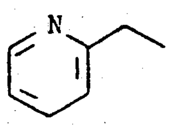 | $CH_3$ | $COOC_2H_5$ | 103 |
| CH | 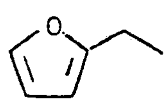 | $CH_3$ | COOtBu | 133 |

Les composés ont été soumis à divers essais pharmacologiques montrant principalement leur activité antagoniste de l'histamine et pour quelques uns de la sérotonine.

1. Activité in vitro : iléon isolé de cobaye

Le test a été effectué selon la méthode de Magnus modifiée par Savini (Arch. Int. Pharmacodyn., 1957, 113, 157), sur des cobayes tricolores mâles pesant environ 300 g, à jeun depuis 18 heures.
Un fragment d'iléon est prélevé, placé à 39°C dans un bain de tyrode traversé par un courant de carbogène ($O_2$ 95 %, $CO_2$ 5 %) et relié à un capteur isotonique avec une tension maximale de 2,5 g. Les contractions sont enregistrées à l'aide d'un microdynamomètre Ugo Basile.
Les contractions sont induites par les divers agents spasmogènes dont la concentration provoquant une réponse submaximale est déterminée (histamine : 1 à $8.10^{-8}$ g/ml).
Les composés de l'invention dissous dans de l'eau distillée ou une solution 0,1 N d'acide méthanesulfonique sont mis en contact avec l'iléon pendant 1 mn avant l'introduction de la substance spasmogène.
Les $CA_{50}$ (concentration diminuant de 50 % les contractions induites par l'histamine) des composés de l'invention vont de $10^{-7}$ à $10^{-8}$ molaire.

2. Activité in vivo : inflammation induite par l'histamine

L'injection intraplantaire dans une des pattes postérieures du rat d'histamine (2 mg) provoque un oedème mesuré, 1 heure après l'injection, à l'aide d'un pléthysmomètre à mercure Ugo Basile.
Les composés de l'invention, mis en suspension dans du tween en solution à 1 % dans de l'eau distillée sont administrés p.o; (0,5 ml/100 g) 1 heure avant l'injection de l'agent inflammatoire.

Les $DA_{40}$ (dose qui diminue de 40 % le volume de l'oedème) des composés de l'invention varient de 0,2 à 10 mg/kg.

Les composés de l'invention sont peu toxiques. Leur DL 50 par voie orale est supérieure à 1000 mg/kg.

Les composés de l'invention peuvent donc être utilisés pour le traitement des allergies telles que allergies respiratoires, allergies cutanées, allergies oculaires et manifestations allergiques diverses.
Certains composés de l'invention sont très sélectifs pour les récepteurs de l'histamine (H1) et sont dénués d'activité anticholinergique et antisérotoninergique aux doses thérapeutiques. Ils possèdent une longue durée d'action et leur disponibilité par voie orale est très élevée.
L'invention comprend, par conséquent, toutes compositions pharmaceutiques renfermant les composés et/ou leurs sels comme principes actifs, en association avec tous excipients appropriés à leur administration par voie orale ou parentérale.

La posologie quotidienne peut aller de 1 à 100 mg par voie orale.

Annexe 1

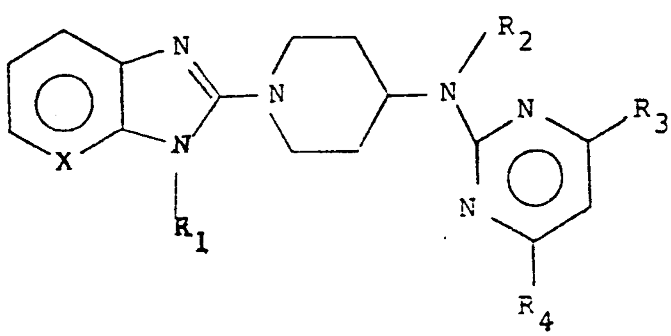

(I)

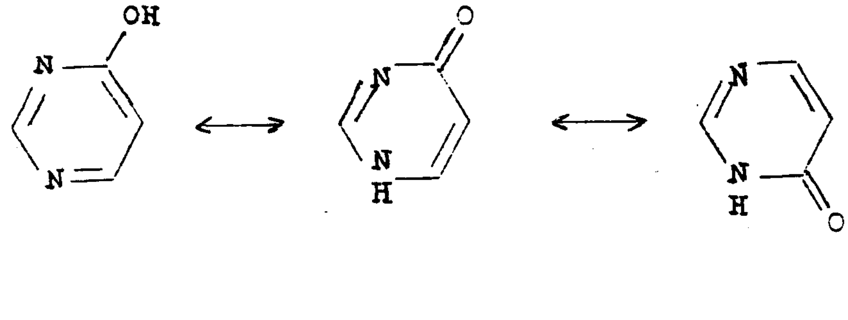

## Annexe 2

Schémas de synthèse

Schéma 1

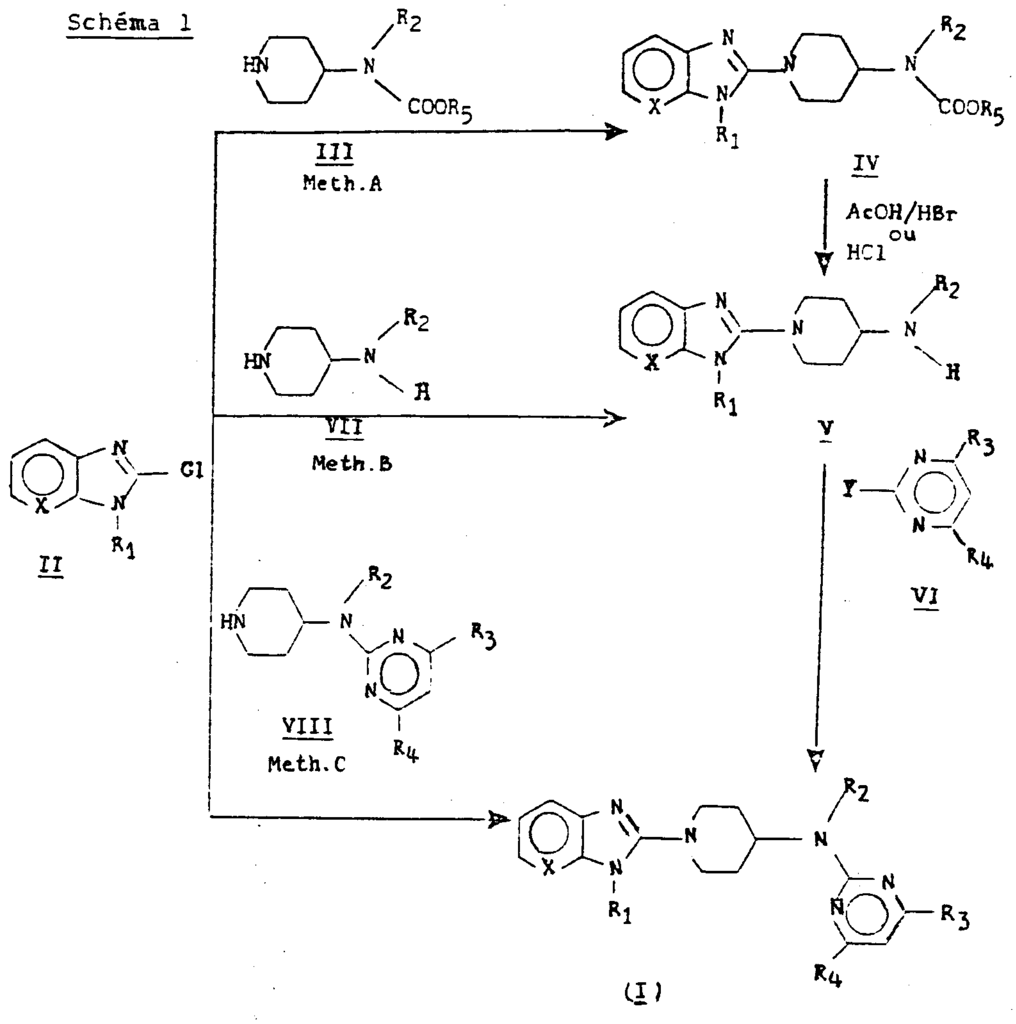


Schéma 2

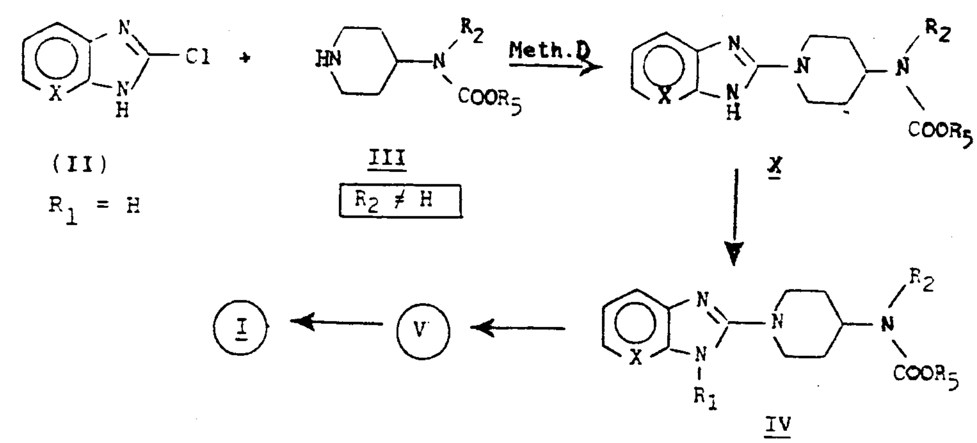

## Revendications

**Revendications pour les Etats contractants suivants : BE, CH, DE, FR, GB, IT, LI, LU, NL, SE**

**1.** Dérivés de benzimidazole répondant à la formule (I)

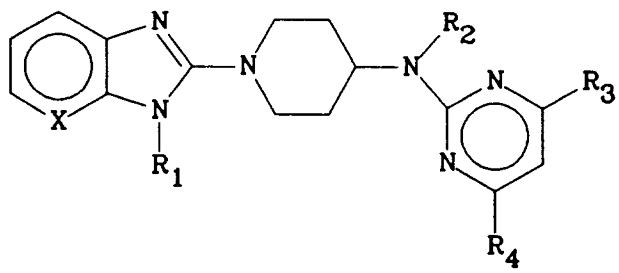

(I)

dans laquelle
X est CH ou N,
$R_1$ est un atome d'hydrogène ou un radical benzyle pouvant porter de 1 à 3 substituants choisis parmi les atomes d'halogène, les radicaux trifluorométhyle, $(C_{1-4})$alkyles, $(C_{1-4})$alcoxy, cyano, méthylthio, méthylsulfinyle et méthylsulfonyle ou encore un radical hétérocycle-méthyle dans lequel l'hétérocycle peut être un radical pyridinyle, thiényle ou furannyle,
$R_2$ est un atome d'hydrogène ou un radical $(C_{1-4})$alkyle,
$R_3$ est un atome d'hydrogène ou le radical hydroxy,
$R_4$ est un atome d'hydrogène ou un radical $(C_{1-4})$alkyle,
éventuellement sous forme de tautomères lorsque $R_3$ est OH, ainsi que leurs sels d'addition aux acides pharmaceutiquement acceptables.

**2.** Dérivés selon la revendication 1, caractérisés par le fait que X est CH ou N, et R est un radical fluoro-4 phénylméthyle, $R_2$, $R_3$ et $R_4$ ayant les significations données dans la revendication 1.

**3.** Dérivés selon la revendication 1, caractérisés par le fait que X est CH.

**4.** Dérivés selon la revendication 3, caractérisés par le fait que $R_1$ est un radical benzyle portant un ou deux substituants tels que définis ci-dessus.

**5.** Dérivés selon la revendication 4, caractérisés par le fait que $R_1$ est un radical benzyle portant en position 4 un atome de fluor ou de chlore ou le radical méthyle, méthoxy, méthylthio, trifluorométhyle, cyano ou méthylsulfinyle.

**6.** Dérivés selon la revendication 5, caractérisés par le fait que $R_1$ est un radical benzyle portant en position 4 un atome de fluor ou le radical méthoxy ou méthylthio.

**7.** Le [[[[(fluoro-4 phényl)méthyl]-1 3H-imidazo [4,5-b] pyridinyl-2]-1 pipéridinyl-4]méthylamino]-2 pyrimidinol-4, selon la revendication 1.

**8.** Le [[[[(fluoro-4 phényl)méthyl]-1 1H-benzimidazolyl-2]-1-pipéridinyl-4]méthylamino]-2 pyrimidinol-4, selon la revendication 1.

**9.** Le [[[[(méthoxy-4 phényl)méthyl]-1 1H-benzimidazolyl-2]-1-pipéridinyl-4]méthylamino]-2 pyrimidinol-4, selon la revendication 1.

**10.** Procédé de préparation des composés selon la revendication 1, procédé caractérisé en ce que l'on fait réagir un composé de formule (II)

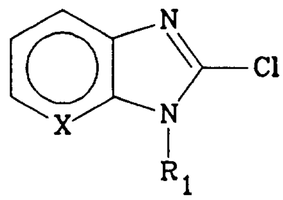

(II)

avec une $(R_2)(R)$amino-4 pipéridine de formule

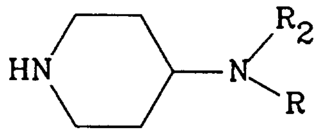

dans laquelle

$R_2$ est H ou $(C_{1-4})$alkyle et R représente un atome d'hydrogène, un groupe $(C_{1-4})$alcoxycarbonyle ou le groupe

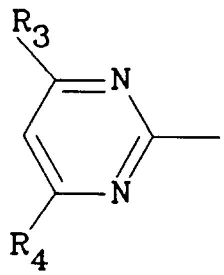

($R_3$ = H ou OH, $R_4$ = H ou $(C_{1-4})$alkyle), et

. lorsque R est H on introduit le groupe pyrimidinyle dans un second temps,

. lorsque R est un groupe $(C_{1-4})$alcoxycarbonyle on élimine ce groupe par hydrolyse puis on introduit le groupe pyrimidinyle.

**11.** Médicament caractérisé en ce qu'il contient un composé tel que spécifié dans l'une quelconque des revendications 1 à 9.

**12.** Composition pharmaceutique caractérisée en ce qu'elle contient un composé tel que spécifié dans l'une quelconque des revendications 1 à 9, en association avec un excipient approprié.

**Revendications pour l'Etat contractant suivant : AT**

1. Procédé de préparation des dérivés de benzimidazole répondant à la formule (I)

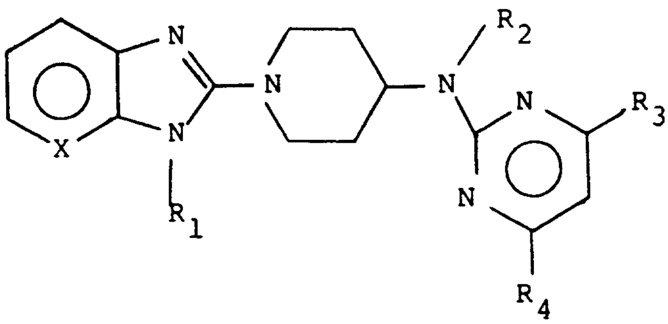

(I)

dans laquelle
X est CH ou N,
$R_1$ est un atome d'hydrogène ou un radical benzyle pouvant porter de 1 à 3 substituants choisis parmi les atomes d'halogène, les radicaux trifluorométhyle, $(C_{1-4})$alkyles, $(C_{1-4})$alcoxy, cyano, méthylthio, méthylsulfinyle et méthylsulfonyle ou encore un radical hétérocycle-méthyle dans lequel l'hétérocycle peut être un radical pyridinyle, thiényle ou furannyle,
$R_2$ est un atome d'hydrogène ou un radical $(C_{1-4})$alkyle,
$R_3$ est un atome d'hydrogène ou le radical hydroxy,
$R_4$ est un atome d'hydrogène ou un radical $(C_{1-4})$alkyle.
éventuellement sous forme de tautomères lorsque $R_3$ est OH, ainsi que de leurs sels d'addition aux acides pharmaceutiquement acceptables,
procédé caractérisé en ce que l'on fait réagir un composé de formule (II)

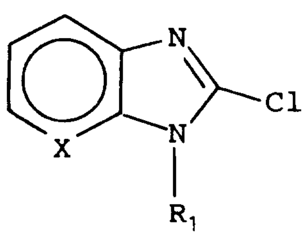

(II)

avec une $(R_2)$(R)amino-4 pipéridine de formule

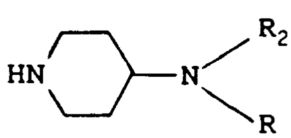

dans laquelle
$R_2$ est H ou $(C_{1-4})$alkyle et
R représente
un atome d'hydrogène
un groupe $(C_{1-4})$alcoxycarbonyle
ou le groupe

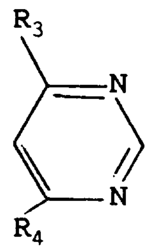

($R_3$ = H ou OH, $R_4$ = H ou ($C_{1-4}$)alkyle), et

. lorsque R est H on introduit le groupe pyrimidinyle dans un second temps,

. lorsque R est un groupe ($C_{1-4}$)alcoxycarbonyle on élimine ce groupe par hydrolyse puis on introduit le groupe pyrimidinyle.

**2.** Procédé de préparation selon la revendication 1, caractérisé en ce que l'on prépare le [[[[(fluoro-4 phényl)méthyl]-1 1H-benzimidazolyl-2]-1 pipéridinyl-4]méthylamino]-2 pyrimidinol-4.

**3.** Procédé de préparation selon la revandication 1, caractérisé en ce que l'on prépare le [[[[(méthoxy-4 phényl)méthyl]-1 1H-benzimidazolyl-2]-1 pipéridinyl-4]méthylamino]-2 pyrimidinol-4.

**4.** Procédé de préparation selon la revendication 1, caractérisé en ce que l'on prépare le [[[[(fluoro-4 phényl)méthyl]-1 3H-imidazo[4,5-b]pyridinyl-2]-1 pipéridinyl-4]methylamino]-2 pyrimidinol-4.

## Claims

**Claims for the following Contracting States : BE, CH, DE, FR, GB, IT, LI, LU, NL, SE**

**1.** Benzimidazole derivatives corresponding to the formula (I)

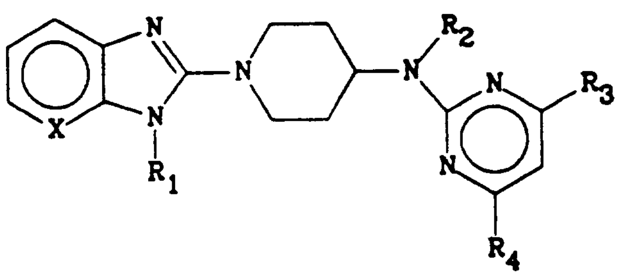

(I)

in which

X is CH or N,

$R_1$ is a hydrogen atom or a benzyl radical which can bear 1 to 3 substituents chosen from halogen atoms and trifluoromethyl, ($C_{1-4}$)alkyl, ($C_{1-4}$)alkoxy, cyano, methylthio, methylsulphinyl and methylsulphonyl radicals, or alternatively a methyl radical bearing a heterocyclic substituent in which the heterocyclic system can be a pyridyl, thienyl or furyl radical,

$R_2$ is a hydrogen atom or a ($C_{1-4}$)alkyl radical,

$R_3$ is a hydrogen atom or a hydroxy radical, and

$R_4$ is a hydrogen atom or a ($C_{1-4}$)alkyl radical,

where appropriate, in tautomeric form when $R_3$ is OH, as well as their addition salts with pharmaceutically acceptable acids.

**2.** Derivatives according to Claim 1, characterized in that X is CH or N, and R is a 4-fluorophenylmethyl radical, $R_2$, $R_3$ and $R_4$ having the meanings given in Claim 1.

**3.** Derivatives according to Claim 1, characterized in that X is CH.

4. Derivatives according to Claim 3, characterized in that $R_1$ is a benzyl radical bearing one or two substituents as defined above.

5. Derivatives according to Claim 4, characterized in that $R_1$ is a benzyl radical bearing at the 4-position a fluorine or chlorine atom or a methyl, methoxy, methylthio, trifluoromethyl, cyano or methylsulphinyl radical.

6. Derivatives according to Claim 5, characterized in that $R_1$ is a benzyl radical bearing at the 4-position a fluorine atom or a methoxy or methylthio radical.

7. 2-[[1-[1-[(4-Fluorophenyl)methyl]-3H-imidazo[4,5-b]-pyrid-2-yl]-4-piperidyl]methylamino]-4-pyrimidinol according to Claim 1.

8. 2-[[1-[1-[(4-Fluorophenyl)methyl]-1H-benzimidazol-2-yl]-4-piperidyl]methylamino]-4-pyrimidinol according to Claim 1.

9. 2-[[1-[1-[(4-Methoxyphenyl)methyl]-1H-benzimidazol-2-yl]-4-piperidyl]methylamino]-4-pyrimidinol according to Claim 1.

10. Process for preparing the compounds according to Claim 1, which process is characterized in that a compound of formula (II)

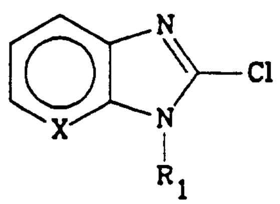

(II)

is reacted with a 4-[($R_2$)(R)amino]piperidine of formula

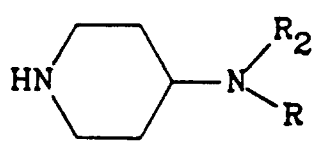

in which
$R_2$ is H or $(C_{1-4})$alkyl and R denotes a hydrogen atom,
a $(C_{1-4})$alkoxycarbonyl group,
or the group

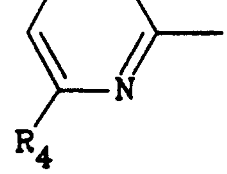

($R_3$ = H or OH, $R_4$ = H or $(C_{1-4})$alkyl), and

- when R is H, the pyrimidinyl group is introduced in a second stage,

- when R is a $(C_{1-4})$alkoxycarbonyl group, this group is removed by hydrolysis and the pyrimidinyl group is then introduced.

**11.** Drug, characterized in that it contains a compound as specified in one of Claims 1 to 9.

**12.** Pharmaceutical composition, characterized in that it contains a compound as specified in any one of Claims 1 to 9, in combination with a suitable excipient.

**Claims for the following Contracting State : AT**

**1.** Process for preparing the benzimidazole derivatives corresponding to the formula (I)

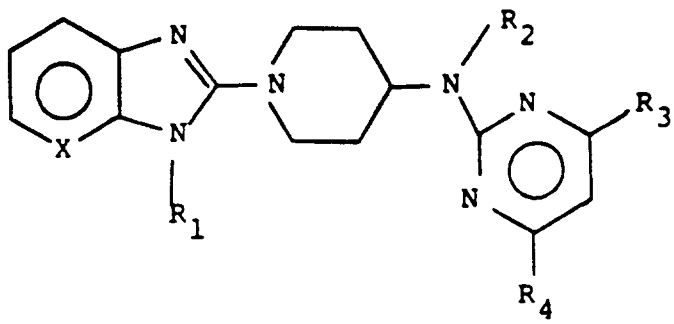

(I)

in which
X is CH or N,
$R_1$ is a hydrogen atom or a benzyl radical which can bear 1 to 3 substituents chosen from halogen atoms and trifluoromethyl, $(C_{1-4})$alkyl, $(C_{1-4})$alkoxy, cyano, methylthio, methylsulphinyl and methylsulphonyl radicals, or alternatively a methyl radical bearing a heterocyclic substituent in which the heterocyclic system can be a pyridyl, thienyl or furyl radical,
$R_2$ is a hydrogen atom or a $(C_{1-4})$alkyl radical,
$R_3$ is a hydrogen atom or a hydroxy radical, and
$R_4$ is a hydrogen atom or a $(C_{1-4})$alkyl radical,
where appropriate, in tautomeric form when $R_3$ is OH, as well as their addition salts with pharmaceutically acceptable acids, which process is characterized in that a compound of formula (II)

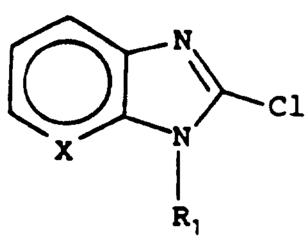

(II)

is reacted with a 4-[$(R_2)$(R)amino]piperidine of formula

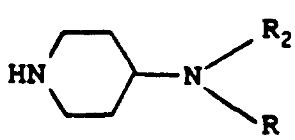

in which
$R_2$ is H or $(C_{1-4})$alkyl and
R denotes

a hydrogen atom,
a $(C_{1-4})$alkoxycarbonyl group,
or the group

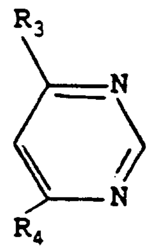

($R_3$ = H or OH, $R_4$ = H or $(C_{1-4})$alkyl), and

- when R is H, the pyrimidinyl group is introduced in a second stage,
- when R is a $(C_{1-4})$alkoxycarbonyl group, this group is removed by hydrolysis and the pyrimidinyl group is then introduced.

**2.** Preparation process according to Claim 1, characterized in that 2-[[1-[1-[(4-fluorophenyl)methyl]-1H-benzimidazol-2-yl]-4-piperidyl]methylamino]-4-pyrimidinol is prepared.

**3.** Preparation process according to Claim 1, characterized in that 2-[[1-[1-[(4-methoxyphenyl)methyl]-1H-benzimidazol-2-yl]-4-piperidyl]methylamino]-4-pyrimidinol is prepared.

**4.** Preparation process according to claim 1, characterized in that 2-[[1-[1-[(4-fluorophenyl)methyl]-3H-imidazo[4,5-b]-pyrid-2-yl]-4-piperidyl]methylamino]-4-pyrimidinol is prepared.

**Patentansprüche**

**Patentansprüche für folgende Vertragsstaaten : BE, CH, DE, FR, GB, IT, LI, LU, NL, SE**

**1.** Benzimidazol-Derivate der Formel (I)

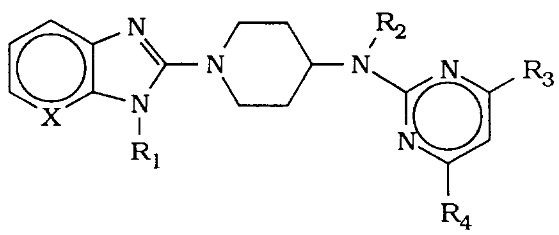

(I)

in der
X CH oder N,
$R_1$ ein Wasserstoffatom oder eine Benzylgruppe, die 1 bis 3 Substituenten tragen kann, ausgewählt aus Halogenatomen, Trifluormethylgruppen, $C_{1-4}$-Alkylgruppen, $C_{1-4}$-Alkoxygruppen, Cyanogruppen, Methylthiogruppen, Methylsulfinylgruppen und Methylsulfonylgruppen, oder eine Heterocyclus-Methylgruppe, worin der Heterocyclus ein Pyridinyl-, Thienyl- oder Furanylrest sein kann, $R_2$ ein Wasserstoffatom oder eine $C_{1-4}$-Alkylgruppe,
$R_3$ ein Wasserstoffatom oder eine Hydroxygruppe und
$R_4$ ein Wasserstoffatom oder eine $C_{1-4}$-Alkylgruppe bedeuten,
gegebenenfalls in Form der Tautomeren, wenn $R_3$ eine OH-Gruppe darstellt, sowie deren Additionssalze mit pharmazeutisch annehmbaren Säuren.

**2.** Derivate nach Anspruch 1, **dadurch gekennzeichnet**, daß X CH oder N und R eine 4-Fluorphenylmethylgruppe bedeuten und $R_2$, $R_3$ und $R_4$ die in Anspruch 1 angegebenen Bedeutungen besitzen.

**3.** Derivate nach Anspruch 1, **dadurch gekennzeichnet**, daß X CH bedeutet.

**4.** Derivate nach Anspruch 3, **dadurch gekennzeichnet**, daß $R_1$ eine Benzylgruppe darstellt, die einen oder zwei der oben definierten Substituenten aufweist.

**5.** Derivate nach Anspruch 4, **dadurch gekennzeichnet**, daß $R_1$ eine Benzylgruppe darstellt, die in der 4-Stellung ein Fluor- oder Chloratom oder eine Methyl-, Methoxy-, Methylthio-, Trifluormethyl-, Cyano- oder Methylsulfinylgruppe aufweist.

**6.** Derivate nach Anspruch 5, **dadurch gekennzeichnet**, daß $R_1$ eine Benzylgruppe darstellt, die in der 4-Stellung ein Fluoratom, eine Methoxygruppe oder eine Methylthiogruppe aufweist.

**7.** 2-[[1-[1-[(4-Fluor-phenyl)-methyl]-3H-imidazo[4,5-b]pyridin-2-yl]-piperidin-4-yl]-methylaminol-pyrimidin-4-ol gemäß Anspruch 1.

**8.** 2-[[1-[1-[(4-Fluor-phenyl)-methyl]-1H-benzimidazol-2-yl]-piperidin-4-yl]-methylamino]-pyrimidin-4-ol nach Anspruch 1.

**9.** 2-[[1-[1-[(4-Methoxy-phenyl)-methyl]-1H-benzimidazol-2-yl]-piperidin-4-yl]-methylamino]-pyrimidin-4-ol gemäß Anspruch 1.

**10.** Verfahren zur Herstellung der Verbindungen nach Anspruch 1, **dadurch gekennzeichnet**, daß man eine Verbindung der Formel (II)

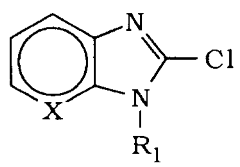

(II)

mit einem $(R_2)(R)$-4-Amino-piperidin der Formel

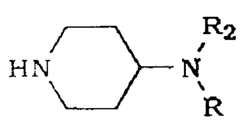

in der
$R_2$ ein Wasserstoffatom oder eine $C_{1-4}$-Alkylgruppe und R ein Wasserstoffatom
oder eine $C_{1-4}$-Alkoxycarbonylgruppe
oder die Gruppe der Formel

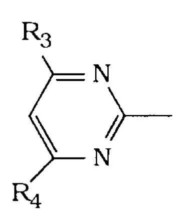

(worin $R_3$ ein Wasserstoffatom oder eine Hydroxylgruppe, $R_4$ ein Wasserstoffatom oder eine $C_{1-4}$-Alkylgruppe darstellen) bedeuten
umsetzt und

. wenn R ein Wasserstoffatom darstellt, in einem zweiten Schritt die Pyrimidinylgruppe einführt,

. wenn R eine $C_{1-4}$-Alkoxycarbonylgruppe darstellt, diese Gruppe durch Hydrolyse eliminiert, und dann die Pyrimidinylgruppe einführt.

**11.** Arzneimittel, **dadurch gekennzeichnet**, daß es eine Verbindung nach einem der Ansprüche 1 bis 9 enthält.

**12.** Pharmazeutische Zubereitung, **dadurch gekennzeichnet**, daß sie eine Verbindung nach einem der Ansprüche 1 bis 9 in Kombination mit einem geeigneten Trägermaterial enthält.

**Patentansprüche für folgenden Vertragsstaat : AT**

**1.** Verfahren zur Herstellung von Benzimidazol-Derivaten der Formel (I)

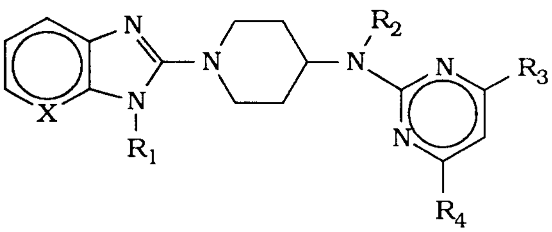

(I)

in der
X CH oder N,
$R_1$ ein Wasserstoffatom oder eine Benzylgruppe, die 1 bis 3 Substituenten tragen kann, ausgewählt aus Halogenatomen, Trifluormethylgruppen, $C_{1-4}$-Alkylgruppen, $C_{1-4}$-Alkoxygruppen, Cyanogruppen, Methylthiogruppen, Methylsulfinylgruppen und Methylsulfonylgruppen, oder eine Heterocyclus-Methylgruppe, worin der Heterocyclus ein Pyridinyl-, Thienyl- oder Furanylrest sein kann,
$R_2$ ein Wasserstoffatom oder eine $C_{1-4}$-Alkylgruppe,
$R_3$ ein Wasserstoffatom oder eine Hydroxygruppe und
$R_4$ ein Wasserstoffatom oder eine $C_{1-4}$-Alkylgruppe bedeuten,
gegebenenfalls in Form der Tautomeren, wenn $R_3$ eine OH-Gruppe darstellt, sowie von deren Additionssalzen mit pharmazeutisch annehmbaren Säuren, **dadurch gekennzeichnet**, daß man eine Verbindung der Formel (II)

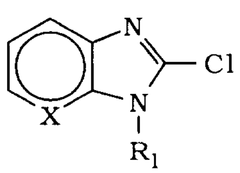

(II)

mit einem ($R_2$)(R)-4-Amino-piperidin der Formel

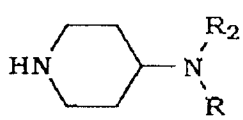

in der
$R_2$ ein Wasserstoffatom oder eine $C_{1-4}$-Alkylgruppe und R ein Wasserstoffatom oder eine $C_{1-4}$-Alkoxycarbonylgruppe
oder die Gruppe der Formel

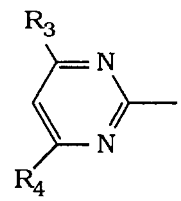

(worin $R_3$ ein Wasserstoffatom oder eine Hydroxylgruppe, $R_4$ ein Wasserstoffatom oder eine $C_{1-4}$-Alkylgruppe darstellen) bedeuten
umsetzt und

- . wenn R ein Wasserstoffatom darstellt, in einem zweiten Schritt die Pyrimidinylgruppe einführt,
- . wenn R eine $C_{1-4}$-Alkoxycarbonylgruppe darstellt, diese Gruppe durch Hydrolyse eliminiert, und dann die Pyrimidinylgruppe einführt.

**2.** Verfahren nach Anspruch 1, **dadurch gekennzeichnet**, daß man 2-[[1-[1-[(4-Fluor-phenyl)-methyl]-1H-benzimidazol-2-yl]-piperidin-4-yl]-methylamino]-pyrimidin-4-ol herstellt.

**3.** Verfahren nach Anspruch 1, **dadurch gekennzeichnet**, daß man 2-[[1-[1-[(4-Methoxy-phenyl)-methyl]-1H-benzimidazol-2-yl]-piperidin-4-yl]-methylamino]-pyrimidin-4-ol herstellt.

**4.** Verfahren nach Anspruch 1, **dadurch gekennzeichnet**, daß man 2-[[1-[1-[(4-Fluor-phenyl)-methyl]-3H-imidazo[4,5-b]pyridin-2-yl]-piperidin-4-yl]-methylamino]-pyrimidin-4-ol herstellt.